# FASCICULE DE BREVET EUROPEEN

(11) **EP 3 315 130 B1**
(45) Date de publication et mention de la délivrance du brevet: **22.03.2023**
(21) Numéro de dépôt: 17199287.8
(22) Date de dépôt: 30.10.2017
(51) Int. Cl.: A61K 31/445, A61K 31/728, A61K 8/73, A61K 9/00, A61P 17/00

(54) **COMPOSITION A BASE D'ACIDE HYALURONIQUE COMPRENANT DE LA MEPIVACAÏNE**
ZUSAMMENSETZUNG AUF HYALURONSÄURE-BASIS, DIE MEPIVACAIN BEINHALTET
HYALURONIC ACID COMPOSITION INCLUDING MEPIVACAINE

(30) Priorité: 28.10.2016 FR 1660522
(43) Date de publication de la demande: 02.05.2018
(73) Titulaire: Laboratoires Vivacy, 75116 Paris (FR)
(72) Inventeur: COUCHOUREL, Denis, 74540 SAINT SYLVESTRE (FR)
(74) Mandataire: Tripoz, Inès

(56) Documents cités:
- WO-A1-2015/015407
- WO-A1-2015/097261
- WO-A2-2010/136585

## Description

L'invention concerne le domaine des gels et hydrogels biodégradables utilisés en tant que biomatériaux et plus particulièrement dans les domaines médicaux et esthétiques.

L'acide hyaluronique est utilisé depuis plus de quinze ans dans le domaine de l'esthétique, où il a prouvé son innocuité et son efficacité. A ce jour, sur le marché des gels de comblement à visée esthétique ou « fillers », les gels à base d'acide hyaluronique réticulé d'origine biofermentaire sont les produits les plus utilisés.

Parmi les applications médicales on citera par exemple les injections pour remplacer les liquides biologiques déficients par exemple dans les articulations pour remplacer le liquide synovial, l'injection suite à une chirurgie pour éviter les adhésions péritonéales, les injections périurétrales pour traiter l'incontinence et les injections suite à une chirurgie de la presbytie.

Parmi les applications esthétiques on citera par exemple les injections pour le comblement des rides, des ridules et des défauts cutanés ou l'augmentation des volumes par exemple les lèvres, les pommettes, etc.

L'utilisation de l'acide hyaluronique d'origine biofermentaire dans les domaines tels que le comblement de rides, la viscosupplémentation, le traitement ophtalmique ou encore le traitement de l'incontinence urinaire est d'autant plus reconnue et appréciée que de par sa présence naturelle dans le corps humain, et plus particulièrement dans le derme, le liquide synovial et le vitré, les risques dus aux effets secondaires sont minimisés.

On appelle « acide hyaluronique », l'acide hyaluronique, réticulé ou non réticulé, seul ou en mélange, éventuellement modifié chimiquement par substitution, seul ou en mélange, éventuellement sous forme de l'un de ses sels, seul ou en mélange.

On appelle « mépivacaïne », la mépivacaïne, ainsi que ses sels et ses isomères isolés.

On appelle « anesthésique local », un anesthésique local ou l'un de ses sels, seuls ou en mélange. En particulier, il peut s'agir de la mépivacaïne, de ses sels et de ses isomères isolés, ou d'autres anesthésiques, comme par exemple la lidocaïne, la bupivacaïne, la benzocaïne, la chloroprocaïne, la procaïne, l'étidocaïne, l'aptocaïne, le chlorobutanol, la diamocaïne, la dyclonine, le guafécaïnol, le polidocanol, l'articaïne, la bupivacaïne, la ropivacaïne, la tétracaïne, la pramocaïne, ou leurs sels et leurs isomères isolés. Dans la présente demande, lorsqu'il est fait allusion à une composition comprenant un anesthésique local, cela signifie que ladite composition comprend de la mépivacaïne et lorsqu'il est fait allusion à une composition comprenant des anesthésiques locaux, cela signifie que ladite composition comprend au moins deux anesthésiques locaux : la mépivacaïne ainsi qu'au moins un autre anesthésique local.

On appelle « sujet demandeur » une personne s'estimant dans la nécessité de se voir administrer une composition à base d'acide hyaluronique par la personne en charge de l'administration.

On appelle « personne en charge de l'administration » une personne réalisant les actes liés au traitement sur le sujet demandeur.

On appelle « composition injectable », une composition susceptible d'être injectée, par exemple avec une aiguille de 27G1/2.

On appelle « Mw » ou « masse moléculaire », la masse moléculaire moyenne en poids des polymères, mesurée en Daltons.

Dans la présente invention, le taux de réticulation X, est défini comme étant égal au rapport suivant : X = (Nombre de moles de réticulant introduites dans le milieu réactionnel) / (Nombre de moles de motif disaccharidique introduites dans le milieu réactionnel).

De façon générale dans le texte de cette demande, les bornes d'un domaine de valeurs sont comprises dans ce domaine, notamment dans l'expression «compris(e(s)) entre ... et ...».

De nombreuses demandes de brevet ou publications ont été déposées ou publiées sur des compositions à base d'acide hyaluronique comprenant, outre l'acide hyaluronique, des actifs ou des excipients pour modifier ou améliorer les propriétés de la composition en fonction des applications particulières.

Par exemple la demande WO 2013/186493 divulgue des compostions d'acide hyaluronique incluant un sucrose octasulfate et la demande WO 2014/032804 divulgue des compositions d'acide hyaluronique incluant un dérivé de vitamine C.

Egalement, des compositions à base d'acide hyaluronique et comprenant un polyol sont décrites dans l'art antérieur.

Dans la demande WO 2007/077399 au nom d'ANTEIS, des compositions à usage dermatologique à base d'acide hyaluronique ou l'un de ses sels et d'un polyol sont présentées. L'ajout de polyol a pour effet de limiter la dégradation des propriétés rhéologiques des compositions lors de la stérilisation à la chaleur humide.

La demande WO 2008/068297 au nom de PIERRE FABRE DERMO COSMETIQUE décrit des effets de protection, notamment in vivo de l'adjonction de mannitol à des compositions d'acide hyaluronique.

La demande WO 2007/128923, au nom d'ANTEIS décrit la préparation d'un gel biocompatible comprenant du glycérol.

La demande WO 2010/136694 au nom d'ANTEIS divulgue une composition injectable à base d'acide hyaluronique réticulé, contenant du glycérol, la composition étant stérilisée par chaleur humide.

Enfin la publication de Belda (Belda et al, 2005, J. Cataract Refract. Surg., 31 : 1213-1218), s'intitulant "Hyaluronic acid combined with mannitol to improve protection against free-radical endothelial damage: Expérimenta) Model", détaille les effets, notamment les effets in vivo, de l'adjonction de mannitol dans des compositions à base d'acide hyaluronique.

Il est par ailleurs connu de rajouter dans les produits de comblement des anesthésiques locaux pour limiter la douleur à l'injection. Cet effet est particulièrement avantageux, l'adjonction dans les compositions à base d'acide hyaluronique d'un composé anesthésique est une condition quasi-obligatoire de son acceptabilité par le sujet demandeur ainsi que par la personne en charge de l'administration.

Certaines demandes de brevet et publications portent ainsi sur des compositions à base d'acide hyaluronique et comprenant de la lidocaïne.

La demande WO 93/12801 au nom de REINMULLER décrit des gels pour traiter les plaies et les cicatrices chéloïdes par injection sous-cutanée. L'exemple 1 de cette demande est relatif à une composition à base d'acide hyaluronique de type « hylagel » (BIOMATRIX), et contenant de la lidocaïne.

L'article de WAHL, (G. Wahl, G., J. Cosme. Dermatol. 7:298-303 (2008)), intitulé « European évaluation of a new hyaluronic acid filler incorporating lidocaine », est relatif à l'incorporation de lidocaïne dans des compositions de comblement à base d'acide hyaluronique. Les résultats présentés sont relatifs à des essais effectués en utilisant JUVEDERM ULTRA^{®} qui est un produit de comblement à base d'acide hyaluronique réticulé. Selon cet article, plus de 87% des patients ont signalé avoir moins mal lors de l'injection de compositions incorporant de la lidocaïne.

La demande FR 2 979 539 au nom de TEOXANE divulgue également une composition à base d'acide hyaluronique comprenant de la lidocaïne. L'intérêt de la mépivacaïne dans cette demande est qu'elle permet, in vitro, de limiter la dégradation de l'acide hyaluronique. Les effets in vivo d'une telle composition à base d'acide hyaluronique et comprenant de la mépivacaïne ne sont pas testés.

Enfin, un certain nombre de produits à base d'acide hyaluronique comprenant de la lidocaïne sont commercialisés, par exemple JUVEDERM^{®}, RESTYLANE^{®}, EMERVEL LIDOCAINE^{®}, TEOSYAL SENSE^{®} etc.

D'autres anesthésiques que la lidocaïne sont également utilisés, par exemple, une demande de brevet EP 2 581 079 au nom de BIOPOLYMER décrit des compositions à base d'acide hyaluronique et de prilocaïne, présentant un profil de relargage rapide de la prilocaïne.

Dans la demande WO 2015/015407 au nom de TEOXANE, des compositions à base d'acide hyaluronique non réticulé et partiellement réticulé comprenant de la mépivacaïne ou de la lidocaïne sont divulguées.

Dans la demande WO 2015/097261 au nom de la demanderesse, des compositions à base d'acide hyaluronique réticulé et non réticulé comprenant de la mépivacaïne sont divulguées. En particulier, il y est mis en évidence qu'in vitro, une composition à base d'acide hyaluronique et comprenant de la mépivacaïne est plus stable.

Dans la demande EP2484387 au nom de Q-MED, des compositions à base d'acide hyaluronique et comprenant un dérivé de vitamine C ainsi qu'un anesthésique local sont présentées. Dans cette demande, l'anesthésique local a un effet de limitation de la dégradation de certaines propriétés rhéologiques lors de l'autoclavage, alors que le dérivé de vitamine C a un effet contraire.

Les anesthésiques locaux sont parfois utilisés en association avec des polyols.

Des compositions à base d'acide hyaluronique comprenant à la fois du mannitol et de la lidocaïne sont commercialisées, c'est par exemple le cas de la gamme STYLAGE^{®} commercialisée par la société demanderesse.

Dans la demande WO 2005/067994 au nom d'ANIKA THERAPEUTICS, des gels à base d'acide hyaluronique qui comprennent notamment de la lidocaïne sont décrits.

Dans la demande WO 2010/015901 au nom d'ALLERGAN, et en particulier dans l'exemple 4, de la lidocaïne est ajoutée à un certain nombre de compositions à base d'acide hyaluronique réticulé.

Dans la demande WO 2010/052430 au nom d'ANTEIS, une composition à base d'acide hyaluronique comprenant un mélange glycérol/sorbitol ainsi que de la lidocaïne est décrite.

Dans la demande WO 2015/097261 au nom de la demanderesse, l'association de mépivacaïne et de polyol dans des compositions à base d'acide hyaluronique est décrite.

En conclusion, l'art antérieur divulgue des compositions à base d'acide hyaluronique pouvant comprendre différents anesthésiques, dont la mépivacaïne dans les demandes WO 2015/097261 et WO 2015/015407 précitées.

Par ailleurs, dans le domaine de l'acide hyaluronique, un certain nombre de difficultés sont rencontrées dès lors que de nombreux composés sont ajoutés. Par exemple, l'étape de stérilisation, qui est le plus souvent (pour différentes raisons) réalisée par autoclavage, aura un effet plus ou moins prononcé sur la rhéologie de la composition, selon que tel ou tel composé additionnel est ajouté. Cette difficulté de prédiction est par exemple illustrée par la demande WO 2012/097272 au nom d'ALLERGAN.

Lors de l'injection de compositions à base d'acide hyaluronique, il est très fréquent d'observer l'apparition d'un oedème qui persiste durant un nombre de jours variable (en fonction des produits utilisés, du sujet demandeur, de la pratique de la personne en charge de l'administration, etc.).

Dans certaines zones précise, par exemple au niveau des lèvres ou du sillon naso-génien, les gonflements sont généralement particulièrement importants, et persistent le plus souvent une bonne semaine.

L'aedème est un gonflement de tissu(s) (tissu conjonctif, tissu du revêtement cutané et/ou tissu mucosal) sous l'effet d'une accumulation de liquide inhabituelle (le plus souvent du sérum sanguin issu du sang).

Evidemment, l'œdème est à distinguer de l'effet volumateur de la composition à base d'acide hyaluronique, ainsi que des fuites vasculaires directes (pouvant par exemple mener à une ecchymose ou à un hématome).

La formation de l'œdème dans le cadre de l'injection d'une composition à base d'acide hyaluronique peut avoir plusieurs causes.

Par exemple, il peut s'agir de l'introduction de l'aiguille ou de la canule, qui provoque une dilacération des tissus : les cellules de l'épiderme, du derme, voire de l'hypoderme sont endommagées, ce qui a pour effet que leurs contenus fuitent dans l'espace intercellulaire, les différentes molécules libérées (issues du contenu des cellules endommagées) induisent une augmentation de la perméabilité vasculaire, notamment au niveau de la microcirculation capillaire. Un filtrat riche en protéines passe alors du compartiment vasculaire vers les tissus interstitiels. La pression osmotique augmente donc, ce qui provoque une diffusion passive du compartiment sanguin vers les tissus interstitiels. Par ailleurs, la plupart des médiateurs issus de la destruction des cellules provoquent aussi une vasodilatation. Tous ces phénomènes ont pour conséquence un gonflement caractéristique de l'œdème. Ce gonflement est bien évidemment différent selon la compétence et la réussite de la personne en charge de l'administration, selon l'attitude de sujet demandeur pendant l'injection, etc.

Outre généralement l'aspect inesthétique de l'œdème, il est particulièrement important de noter que dans le cadre d'une injection d'acide hyaluronique, ce dernier peut mener à une mauvaise appréciation de l'effet de comblement par la personne en charge de l'administration. Ainsi, une trop faible quantité d'acide hyaluronique pourra avoir été injectée, créant ainsi un comblement insuffisant. Plus gênant, l'acide hyaluronique pourra avoir été inégalement réparti, créant ainsi un aspect inesthétique inverse de l'unique but (esthétique) poursuivi.

En conclusion, la prévention dans l'œdème dans le cadre d'injection d'acide hyaluronique est purement guidée par un effet esthétique / cosmétique recherché. Il n'est nullement question dans ce cas d'aspects thérapeutiques. Par exemple, il va sans dire que la vie du sujet demandeur n'est nullement en danger. Si l'administration d'acide hyaluronique ne peut pas être considérée comme thérapeutique, l'oedème peut être considéré comme un véritable effet secondaire lié à cette administration non thérapeutique.

La demande WO 2010/136585 au nom de GALDERMA est relative à des compositions à base d'acide hyaluronique, comprenant de la brimonidine, ce composé permettant selon le demandeur de réduire des effets secondaires dus à l'injection, par exemple - entres autres - les oedèmes et les ecchymoses. Dans cette demande, il est donc prévu l'adjonction d'un composé additionnel ayant pour but unique la prévention de certains effets secondaires.

Il existe un besoin à l'heure actuelle de disposer d'une composition à base d'acide hyaluronique, véritablement susceptible d'être injectée, et :
- susceptible de limiter la douleur lors de l'injection ;
- comportant un minimum de constituants ;
- susceptible de limiter l'occurrence, l'apparition et la persistance des oedèmes.

De manière surprenante, il a été mis en évidence que l'inclusion, dans une composition à base d'acide hyaluronique, de mépivacaïne, permettait de réduire l'occurrence, l'apparition et la persistance des oedèmes.

De manière encore plus surprenante, il a été mis en évidence que l'inclusion, dans une composition à base d'acide hyaluronique, de mépivacaïne en association avec un polyol (préférentiellement choisi dans le groupe constitué du mannitol et du sorbitol, et plus préférentiellement le mannitol), permettait de réduire encore l'occurrence, l'apparition et la persistance des oedèmes.

L'invention est exclusivement constituée par l'objet des revendications L'invention concerne la mépivacaïne pour utilisation dans la prévention de la formation des oedèmes induits par une composition aqueuse comprenant au moins un acide hyaluronique, ladite composition comprenant ladite mépivacaïne.

Les oedèmes peuvent notamment être induits par ladite composition.

Dans un mode de réalisation, la composition destinée à être utilisée selon l'invention est caractérisée en ce qu'elle est destinée à être utilisée dans la prévention de la formation des oedèmes induits par le geste de la personne en charge de l'administration.

Dans un mode de réalisation, la composition destinée à être utilisée selon l'invention est caractérisée en ce qu'elle est destinée à être utilisée dans la prévention de la formation des oedèmes induits par la présence de ladite composition dans l'organisme.

Dans un mode de réalisation, la composition destinée à être utilisée selon l'invention est caractérisée en ce que ladite composition n'est pas une composition thérapeutique.

Dans un mode de réalisation, l'oedème est un effet secondaire de l'administration de ladite composition aqueuse comprenant au moins un acide hyaluronique et de la mépivacaïne.

Dans un mode de réalisation, l'oedème est situé au niveau du sillon naso-génien.

Dans un mode de réalisation, la composition destinée à être utilisée selon l'invention est caractérisée en ce qu'elle est non-thérapeutique.

Dans un mode de réalisation, la composition destinée à être utilisée selon l'invention est caractérisée en ce que ladite utilisation est non-thérapeutique.

Dans un mode de réalisation, la composition destinée à être utilisée selon l'invention est caractérisée en ce que ladite utilisation est esthétique.

Dans un mode de réalisation, la composition destinée à être utilisée selon l'invention est caractérisée en ce que ladite utilisation est cosmétique.

Dans un mode de réalisation, la composition destinée à être utilisée selon l'invention est caractérisée en ce que la masse moléculaire Mw de l'au moins un acide hyaluronique est comprise dans un intervalle de 0,01 MDa et 5 MDa.

Dans un mode de réalisation, la composition destinée à être utilisée selon l'invention est caractérisée en ce que la masse moléculaire Mw de l'au moins un acide hyaluronique est comprise dans un intervalle de 0,1 MDa et 3,5 MDa.

Dans un mode de réalisation, la composition destinée à être utilisée selon l'invention est caractérisée en ce que la masse moléculaire Mw de l'au moins un acide hyaluronique est comprise dans un intervalle de 1 MDa et 3 MDa.

Dans un mode de réalisation, la composition destinée à être utilisée selon l'invention est caractérisée en ce que la masse moléculaire Mw de l'au moins un acide hyaluronique est comprise dans un intervalle de 1 MDa et 2 MDa.

Dans un mode de réalisation, la composition destinée à être utilisée selon l'invention est caractérisée en ce que la masse moléculaire Mw de l'au moins un acide hyaluronique est de 1 MDa.

Dans un mode de réalisation, la composition destinée à être utilisée selon l'invention est caractérisée en ce que la masse moléculaire Mw de l'au moins un acide hyaluronique est de 2 MDa.

Dans un mode de réalisation, la composition destinée à être utilisée selon l'invention est caractérisée en ce que la masse moléculaire Mw de l'au moins un acide hyaluronique est de 3 MDa.

Dans un mode de réalisation, la composition destinée à être utilisée selon l'invention est caractérisée en ce qu'elle comprend au moins un acide hyaluronique modifié chimiquement par substitution, réticulé ou non réticulé, ou l'un de ses sels, seul ou en mélange.

Dans un mode de réalisation, la composition destinée à être utilisée selon l'invention est caractérisée en ce qu'elle comprend au moins un acide hyaluronique, ou l'un de ses sels, substitué par un groupement apportant des propriétés lipophile ou hydratante, comme par exemple les acides hyaluroniques substitués tels que décrits dans la demande de brevet FR 2 983 483 au nom de la demanderesse.

Dans un mode de réalisation, la composition destinée à être utilisée selon l'invention est caractérisée en ce qu'au moins un acide hyaluronique est sous forme de sel de sodium ou de potassium.

Dans un mode de réalisation, la composition destinée à être utilisée selon l'invention est caractérisée en ce que la concentration en l'au moins un acide hyaluronique [HA] est comprise entre 2 mg/g et 50 mg/g de poids total de ladite composition.

Dans un mode de réalisation, la composition destinée à être utilisée selon l'invention est caractérisée en ce que la concentration en l'au moins un acide hyaluronique [HA] est comprise entre 4 mg/g et 40 mg/g de poids total de ladite composition.

Dans un mode de réalisation, la composition destinée à être utilisée selon l'invention est caractérisée en ce que la concentration en l'au moins un acide hyaluronique [HA] est comprise entre 5 mg/g et 30 mg/g de poids total de ladite composition.

Dans un mode de réalisation, la composition destinée à être utilisée selon l'invention est caractérisée en ce que la concentration en l'au moins un acide hyaluronique [HA] est comprise entre 10 mg/g et 30 mg/g de poids total de ladite composition.

Dans un mode de réalisation, la composition destinée à être utilisée selon l'invention est caractérisée en ce que la concentration en l'au moins un acide hyaluronique [HA] est de 20 mg/g de poids total de ladite composition.

Dans un mode de réalisation, la composition destinée à être utilisée selon l'invention est caractérisée en ce que la concentration en l'au moins un acide hyaluronique est comprise entre 0,2 et 5% en poids par rapport au poids total de ladite composition.

Dans un mode de réalisation, la composition destinée à être utilisée selon l'invention est caractérisée en ce que la concentration en l'au moins un acide hyaluronique est supérieure ou égale à 1 % en poids par rapport au poids total de ladite composition.

Dans un mode de réalisation, la composition destinée à être utilisée selon l'invention est caractérisée en ce que la concentration en l'au moins un acide hyaluronique est inférieure ou égale à 3 % en poids par rapport au poids total de ladite composition.

Dans un mode de réalisation, la composition destinée à être utilisée selon l'invention est caractérisée en ce que la concentration en mépivacaïne [MEPI] est comprise entre 0,01 mg/g et 50 mg/g de poids total de ladite composition.

Dans un mode de réalisation, la composition destinée à être utilisée selon l'invention est caractérisée en ce que la concentration en mépivacaïne [MEPI] est comprise entre 0,05 mg/g et 45 mg/g de poids total de ladite composition.

Dans un mode de réalisation, la composition destinée à être utilisée selon l'invention est caractérisée en ce que la concentration en mépivacaïne [MEPI] est comprise entre 0,1 mg/g et 40 mg/g de poids total de ladite composition.

Dans un mode de réalisation, la composition destinée à être utilisée selon l'invention est caractérisée en ce que la concentration en mépivacaïne [MEPI] est comprise entre 0,2 mg/g et 30 mg/g de poids total de ladite composition.

Dans un mode de réalisation, la composition destinée à être utilisée selon l'invention est caractérisée en ce que la concentration en mépivacaïne [MEPI] est comprise entre 0,5 mg/g et 20 mg/g de poids total de ladite composition.

Dans un mode de réalisation, la composition destinée à être utilisée selon l'invention est caractérisée en ce que la concentration en mépivacaïne [MEPI] est comprise entre 1 mg/g et 15 mg/g de poids total de ladite composition.

Dans un mode de réalisation, la composition destinée à être utilisée selon l'invention est caractérisée en ce que la concentration en mépivacaïne [MEPI] est comprise entre 1 mg/g et 10 mg/g de poids total de ladite composition.

Dans un mode de réalisation, la composition destinée à être utilisée selon l'invention est caractérisée en ce que la concentration en mépivacaïne [MEPI] est comprise entre 1 mg/g et 6 mg/g de poids total de ladite composition.

Dans un mode de réalisation, la composition destinée à être utilisée selon l'invention est caractérisée en ce que la concentration en mépivacaïne [MEPI] est comprise entre 1 mg/g et 5 mg/g de poids total de ladite composition.

Dans un mode de réalisation, la composition destinée à être utilisée selon l'invention est caractérisée en ce que la concentration en mépivacaïne [MEPI] est comprise entre 2 mg/g et 5 mg/g de poids total de ladite composition.

Dans un mode de réalisation, la composition destinée à être utilisée selon l'invention est caractérisée en ce que la concentration en mépivacaïne [MEPI] est comprise entre 6 mg/g et 10 mg/g de poids total de ladite composition.

Dans un mode de réalisation, la composition destinée à être utilisée selon l'invention est caractérisée en ce que la concentration en mépivacaïne [MEPI] est de 1 mg/g de poids total de ladite composition.

Dans un mode de réalisation, la composition destinée à être utilisée selon l'invention est caractérisée en ce que la concentration en mépivacaïne [MEPI] est de 3 mg/g de poids total de ladite composition.

Dans un mode de réalisation, la composition destinée à être utilisée selon l'invention est caractérisée en ce que concentration en mépivacaïne [MEPI] est de 4 mg/g de poids total de ladite composition.

Dans un mode de réalisation, la composition destinée à être utilisée selon l'invention est caractérisée en ce que la concentration en mépivacaïne [MEPI] est de 5 mg/g de poids total de ladite composition.

Dans un mode de réalisation, la composition destinée à être utilisée selon l'invention est caractérisée en ce que la concentration en mépivacaïne [MEPI] est de 6 mg/g de poids total de ladite composition.

Dans un mode de réalisation, la composition destinée à être utilisée selon l'invention est caractérisée en ce que la concentration en mépivacaïne [MEPI] est de 10 mg/g de poids total de ladite composition.

Dans un mode de réalisation, la composition destinée à être utilisée selon l'invention est caractérisée en ce que le ratio massique entre la concentration en l'au moins un acide hyaluronique [HA] et la concentration en mépivacaïne [MEPI] ; [HA] / [MEPI] est compris entre 0,1 et 50 ; 0,1 ≤ [HA] / [MEPI] ≤ 50.

Dans un mode de réalisation, la composition destinée à être utilisée selon l'invention est caractérisée en ce que le ratio massique entre la concentration en l'au moins un acide hyaluronique [HA] et la concentration en mépivacaïne [MEPI] : [HA] / [MEPI] est compris entre 0,5 et 40, 0,5 ≤ [HA] / [MEPI] ≤ 40.

Dans un mode de réalisation, la composition destinée à être utilisée selon l'invention est caractérisée en ce que le ratio massique entre la concentration en l'au moins un acide hyaluronique [HA] et la concentration en mépivacaïne [MEPI] : [HA] / [MEPI] est compris entre 1 et 30, 1 ≤ [HA] / [MEPI] ≤ 30.

Dans un mode de réalisation, la composition destinée à être utilisée selon l'invention est caractérisée en ce que le ratio massique entre la concentration en l'au moins un acide hyaluronique [HA] et la concentration en mépivacaïne [MEPI] : [HA] / [MEPI] est compris entre 2 et 20, 2 ≤ [HA] / [MEPI] ≤ 20.

Dans un mode de réalisation, la composition destinée à être utilisée selon l'invention est caractérisée en ce que le ratio massique entre la concentration en l'au moins un acide hyaluronique [HA] et la concentration en mépivacaïne [MEPI] : [HA] / [MEPI] est compris entre 7 / 3 et 26 / 3, 7 / 3 ≤ [HA] / [MEPI] ≤ 26 / 3.

Dans un mode de réalisation, la composition destinée à être utilisée selon l'invention est caractérisée en ce que le ratio massique entre la concentration en l'au moins un acide hyaluronique [HA] et la concentration en mépivacaïne [MEPI] : [HA] / [MEPI] est compris entre 2 et 20 / 3, 2 ≤ [HA] / [MEPI] ≤ 20 / 3.

Dans un mode de réalisation, la composition destinée à être utilisée selon l'invention est caractérisée en ce que le ratio massique entre la concentration en l'au moins un acide hyaluronique [HA] et la concentration en mépivacaïne [MEPI] : [HA] / [MEPI] est compris entre 2 et 10 / 3, 2 ≤ [HA] / [MEPI] ≤ 10 / 3.

Dans un mode de réalisation, la composition destinée à être utilisée selon l'invention est caractérisée en ce que le ratio massique entre la concentration en l'au moins un acide hyaluronique [HA] et la concentration en mépivacaïne [MEPI] : [HA] / [MEPI] est de 20.

Dans un mode de réalisation, la composition destinée à être utilisée selon l'invention est caractérisée en ce que le ratio massique entre la concentration en l'au moins un acide hyaluronique [HA] et la concentration en mépivacaïne [MEPI] : [HA] / [MEPI] est de 26 / 3.

Dans un mode de réalisation, la composition destinée à être utilisée selon l'invention est caractérisée en ce que le ratio massique entre la concentration en l'au moins un acide hyaluronique [HA] et la concentration en mépivacaïne [MEPI] : [HA] / [MEPI] est de 20 / 3.

Dans un mode de réalisation, la composition destinée à être utilisée selon l'invention est caractérisée en ce que le ratio massique entre la concentration en l'au moins un acide hyaluronique [HA] et la concentration en mépivacaïne [MEPI] : [HA] / [MEPI] est de 10 / 3.

Dans un mode de réalisation, la composition destinée à être utilisée selon l'invention est caractérisée en ce que le ratio massique entre la concentration en l'au moins un acide hyaluronique [HA] et la concentration en mépivacaïne [MEPI] : [HA] / [MEPI] est de 7 / 3.

Dans un mode de réalisation, la composition destinée à être utilisée selon l'invention est caractérisée en ce que le ratio massique entre la concentration en l'au moins un acide hyaluronique [HA] et la concentration en mépivacaïne [MEPI] : [HA] / [MEPI] est de 2.

Dans un mode de réalisation, la composition destinée à être utilisée selon l'invention est caractérisée en ce qu'elle comprend au moins un acide hyaluronique non réticulé ou l'un de ses sels, seul ou en mélange.

Dans un mode de réalisation, la composition destinée à être utilisée selon l'invention est caractérisée en ce qu'elle comprend au moins 5% d'acide hyaluronique non réticulé ou l'un de ses sels, seul ou en mélange.

Dans un mode de réalisation, la composition destinée à être utilisée selon l'invention est caractérisée en ce qu'elle comprend au moins 15% d'acide hyaluronique non réticulé ou l'un de ses sels, seul ou en mélange.

Dans un mode de réalisation, la composition destinée à être utilisée selon l'invention est caractérisée en ce qu'elle comprend au moins 30% d'acide hyaluronique non réticulé ou l'un de ses sels, seul ou en mélange.

Dans un mode de réalisation, la composition destinée à être utilisée selon l'invention est caractérisée en ce qu'elle comprend au moins 45% d'acide hyaluronique non réticulé ou l'un de ses sels, seul ou en mélange.

Dans un mode de réalisation, la composition destinée à être utilisée selon l'invention est caractérisée en ce qu'elle comprend au moins 75% d'acide hyaluronique non réticulé ou l'un de ses sels, seul ou en mélange.

Dans un mode de réalisation, la composition destinée à être utilisée selon l'invention est caractérisée en ce qu'elle comprend au moins 90% d'acide hyaluronique non réticulé ou l'un de ses sels, seul ou en mélange.

Dans un mode de réalisation, la composition destinée à être utilisée selon l'invention est caractérisée en ce qu'elle comprend un mélange d'acides hyaluroniques, non réticulés.

Dans un mode de réalisation, la composition destinée à être utilisée selon l'invention est caractérisée en ce qu'elle comprend un mélange d'acides hyaluroniques, ou l'un de leurs sels, non réticulés, ayant des masses moléculaires différentes.

Dans un mode de réalisation, la composition destinée à être utilisée selon l'invention est caractérisée en ce qu'elle comprend au moins un acide hyaluronique réticulé ou l'un de ses sels, seul ou en mélange.

Dans un mode de réalisation, la composition destinée à être utilisée selon l'invention est caractérisée en ce qu'elle comprend au moins 5% d'acide hyaluronique réticulé ou l'un de ses sels, seul ou en mélange.

Dans un mode de réalisation, la composition destinée à être utilisée selon l'invention est caractérisée en ce qu'elle comprend au moins 15% d'acide hyaluronique réticulé ou l'un de ses sels, seul ou en mélange.

Dans un mode de réalisation, la composition destinée à être utilisée selon l'invention est caractérisée en ce qu'elle comprend au moins 30% d'acide hyaluronique réticulé ou l'un de ses sels, seul ou en mélange.

Dans un mode de réalisation, la composition destinée à être utilisée selon l'invention est caractérisée en ce qu'elle comprend au moins 45% d'acide hyaluronique réticulé ou l'un de ses sels, seul ou en mélange.

Dans un mode de réalisation, la composition destinée à être utilisée selon l'invention est caractérisée en ce qu'elle comprend au moins 75% d'acide hyaluronique réticulé ou l'un de ses sels, seul ou en mélange.

Dans un mode de réalisation, la composition destinée à être utilisée selon l'invention est caractérisée en ce qu'elle comprend au moins 90% d'acide hyaluronique réticulé ou l'un de ses sels, seul ou en mélange.

Dans un mode de réalisation, la composition destinée à être utilisée selon l'invention est caractérisée en ce que l'au moins un acide hyaluronique réticulé présente un taux de réticulation X compris entre 0,001 et 0,5.

Dans un mode de réalisation, la composition destinée à être utilisée selon l'invention est caractérisée en ce que l'au moins un acide hyaluronique réticulé présente un taux de réticulation X compris entre 0,01 et 0,4.

Dans un mode de réalisation, la composition destinée à être utilisée selon l'invention est caractérisée en ce que l'au moins un acide hyaluronique réticulé présente un taux de réticulation X compris entre 0,1 et 0,3.

Dans un mode de réalisation, la composition destinée à être utilisée selon l'invention est caractérisée en ce que l'au moins un acide hyaluronique réticulé présente un taux de réticulation X de 0,06.

Dans un mode de réalisation, la composition destinée à être utilisée selon l'invention est caractérisée en ce que l'au moins un acide hyaluronique réticulé présente un taux de réticulation X de 0,07.

Dans un mode de réalisation, la composition destinée à être utilisée selon l'invention est caractérisée en ce que l'au moins un acide hyaluronique réticulé présente un taux de réticulation X de 0,12.

Dans un mode de réalisation, la composition destinée à être utilisée selon l'invention est caractérisée en ce que l'au moins un acide hyaluronique réticulé est auto-réticulé.

Dans le cadre de la présente demande, on appelle « acide hyaluronique auto-réticulé » un acide hyaluronique réticulé sans présence de groupement intermédiaire.

Dans un mode de réalisation, la composition destinée à être utilisée selon l'invention est caractérisée en ce que la réticulation est réalisée au moyen d'au moins un agent de réticulation.

Dans un mode de réalisation, la composition destinée à être utilisée selon l'invention est caractérisée en ce que la réticulation est réalisée au moyen d'au moins un agent de réticulation bi- ou polyfonctionnel.

Dans un mode de réalisation, la composition destinée à être utilisée selon l'invention est caractérisée en ce que la réticulation est réalisée au moyen d'au moins un agent de réticulation bi- ou polyfonctionnel choisi dans le groupe constitué par l'éther d'éthylèneglycoldiglycidyle, l'éther de butanedioldiglycidyle (BDDE), l'éther de polyglycérolpolyglycidyle, l'éther de polyéthylèneglycoldiglycidyle, l'éther de polypropylèneglycoldiglycidyle, un bis- ou polyépoxy tel que 1,2,3,4-diépoxybutane ou 1,2,7,8-diépoxyoctane, une dialkylsulfone, la divinylsulfone, le formaldéhyde, l'épichlorohydrine ou bien encore le glutaraldéhyde, les carbodiimides tels que par exemple le 1-éthyl-3-[3-dimethylaminopropyl]carbodiimide hydrochloride (EDC).

Dans un mode de réalisation, la composition destinée à être utilisée selon l'invention est caractérisée en ce que la réticulation est réalisée au moyen de l'éther de butanedioldiglycidyle (BDDE) ou du 1,2,7,8-diépoxyoctane.

Dans un mode de réalisation, la composition destinée à être utilisée selon l'invention est caractérisée en ce que la réticulation est réalisée au moyen de l'éther de butanedioldiglycidyle (BDDE).

Dans un mode de réalisation, la composition destinée à être utilisée selon l'invention est caractérisée en ce qu'elle comprend au moins un acide hyaluronique doublement réticulé tel que décrit dans la demande de brevet WO 2000 / 046253 au nom de Fermentech Medical Limited.

Dans un mode de réalisation, le mélange d'acides hyaluroniques, ou l'un de leurs sels, réticulés est un mélange obtenu par mélange de plusieurs acides hyaluroniques, ou l'un de leurs sels, de masses moléculaires différentes préalablement à leur réticulation, tel que décrit dans la demande de brevet WO 2004 / 092222 au nom de Cornéal Industrie.

Dans un mode de réalisation, la composition destinée à être utilisée selon l'invention est caractérisée en ce qu'elle comprend au moins un acide hyaluronique coréticulé ou l'un de ses sels, seul ou en mélange.

Dans un mode de réalisation, la composition destinée à être utilisée selon l'invention est caractérisée en ce qu'elle comprend un mélange d'acides hyaluroniques, réticulé(s) et non réticulé(s).

Dans un mode de réalisation, la composition destinée à être utilisée selon l'invention est caractérisée en ce qu'elle comprend un mélange d'acides hyaluroniques, réticulés.

Dans un mode de réalisation, le mélange d'acides hyaluroniques, ou l'un de leurs sels, réticulés est un mélange monophasique tel que celui décrit dans la demande de brevet WO 2009 / 071697 au nom de la demanderesse.

Dans un mode de réalisation, le mélange d'acides hyaluroniques, ou l'un de leurs sels, réticulés est un mélange monophasique de plusieurs acides hyaluroniques réticulés, tel que celui décrit dans la demande de brevet WO 2009 / 071697 au nom de la demanderesse, selon les modes de réalisation suivants :

Dans un mode de réalisation, le mélange est constitué de deux acides hyaluroniques réticulés (un premier acide hyaluronique et un second acide hyaluronique).

Dans un mode de réalisation, le premier acide hyaluronique a une concentration en acide hyaluronique comprise entre 2 et 50 mg/ml, préférentiellement comprise entre 5 et 30 mg/ml, préférentiellement comprise entre 6 et 15 mg/ml, et le second acide hyaluronique a une concentration en acide hyaluronique comprise entre 2 et 50 mg/ml, préférentiellement comprise entre 5 et 30 mg/ml, préférentiellement comprise entre 6 et 15 mg/ml.

Dans un mode de réalisation, le premier acide hyaluronique a un taux de réticulation compris entre 0,05 et 0,15, préférentiellement compris entre 0,07 et 0,14, préférentiellement compris entre 0,09 et 0,12, et le second acide hyaluronique a un taux de réticulation compris entre 0,05 et 0,15, préférentiellement compris entre 0,07 et 0,12, préférentiellement compris entre 0,07 et 0,10.

Dans un mode de réalisation, le premier acide hyaluronique a une masse moléculaire comprise entre 0,8 et 1,5 MDa ou comprise entre 2,5 et 3,5 MDa, et le second acide hyaluronique a une masse moléculaire comprise entre 0,8 et 1,5 MDa ou comprise entre 2,5 et 3,5 MDa.

Dans un mode de réalisation, le premier acide hyaluronique a une masse moléculaire comprise entre 0,8 et 1,5 MDa, et le second acide hyaluronique a une masse moléculaire comprise entre 2,5 et 3,5 MDa.

Dans un mode de réalisation, le premier acide hyaluronique a une masse moléculaire comprise entre 2,5 et 3,5 MDa, et le second acide hyaluronique a une masse moléculaire comprise entre 0,8 et 1,5 MDa.

Dans un mode de réalisation, les proportions entre ledit premier et ledit second acide hyaluronique sont comprises entre 40/60 et 60/40.

Dans un mode de réalisation, le premier acide hyaluronique a une concentration en acide hyaluronique comprise entre 2 et 50 mg/ml, préférentiellement comprise entre 5 et 30 mg/ml, préférentiellement comprise entre 6 et 15 mg/ml, un taux de réticulation compris entre 0,05 et 0,15, préférentiellement compris entre 0,07 et 0,14, préférentiellement compris entre 0,09 et 0,12, et une masse moléculaire comprise entre 0,8 et 1,5 MDa ou comprise entre 2,5 et 3,5 MDa, et le second acide hyaluronique a une concentration en acide hyaluronique comprise entre 2 et 50 mg/ml, préférentiellement comprise entre 5 et 30 mg/ml, préférentiellement comprise entre 6 et 15 mg/ml, un taux de réticulation compris entre 0,05 et 0,15, préférentiellement compris entre 0,07 et 0,12, préférentiellement compris entre 0,07 et 0,10, et une masse moléculaire comprise entre 0,8 et 1,5 MDa ou comprise entre 2,5 et 3,5 MDa, les proportions entre ledit premier et ledit second acide hyaluronique étant comprises entre 40/60 et 60/40.

Dans un mode de réalisation préféré, le premier acide hyaluronique a une masse moléculaire comprise entre 0,8 et 1,5 MDa et le second acide hyaluronique a une masse moléculaire comprise entre 2,5 et 3,5 MDa. Dans un mode de réalisation préféré, le second acide hyaluronique a une masse moléculaire comprise entre 0,8 et 1,5 MDa et le premier acide hyaluronique a une masse moléculaire comprise entre 2,5 et 3,5 MDa.

Dans un mode de réalisation, la composition destinée à être utilisée selon l'invention est caractérisée en ce qu'elle comprend un mélange d'acides hyaluroniques, ou l'un de leurs sels, réticulé et non réticulé.

Dans un mode de réalisation, la composition destinée à être utilisée selon l'invention est caractérisée en ce qu'elle comprend un mélange d'acides hyaluroniques, ou l'un de leurs sels, réticulés.

Dans un mode de réalisation, la composition destinée à être utilisée selon l'invention est caractérisée en ce qu'elle comprend en outre au moins un polyol.

Dans un mode de réalisation, la composition destinée à être utilisée selon l'invention est caractérisée en ce que l'au moins un polyol est choisi dans le groupe constitué par le glycérol, le sorbitol, le propylène glycol, le xylitol, le mannitol, l'érythritol, le maltitol et le lactitol, seul ou en mélange.

Dans un mode de réalisation, la composition destinée à être utilisée selon l'invention est caractérisée en ce que l'au moins un polyol est choisi dans le groupe constitué par le mannitol, le sorbitol, le maltitol et le glycérol, seul ou en mélange.

Dans un mode de réalisation, la composition destinée à être utilisée selon l'invention est caractérisée en ce que l'au moins un polyol est choisi dans le groupe constitué par le mannitol, le sorbitol et le maltitol, seul ou en mélange.

Dans un mode de réalisation, la composition destinée à être utilisée selon l'invention est caractérisée en ce que l'au moins un polyol est le mannitol.

Dans un mode de réalisation, la composition destinée à être utilisée selon l'invention est caractérisée en ce que l'au moins un polyol est le sorbitol.

Dans un mode de réalisation, la composition destinée à être utilisée selon l'invention est caractérisée en ce que l'au moins un polyol est le maltitol.

Dans un mode de réalisation, la composition destinée à être utilisée selon l'invention est caractérisée en ce que l'au moins un polyol est le glycérol.

Dans un mode de réalisation, la composition destinée à être utilisée selon l'invention est caractérisée en ce qu'elle comprend au moins du mannitol et du sorbitol.

Dans un mode de réalisation, la composition destinée à être utilisée selon l'invention est caractérisée en ce qu'elle comprend au moins du mannitol et du maltitol.

Dans un mode de réalisation, la composition destinée à être utilisée selon l'invention est caractérisée en ce que la concentration en l'au moins un polyol [Po] est comprise entre 0,01 mg/g et 50 mg/g.

Dans un mode de réalisation, la composition destinée à être utilisée selon l'invention est caractérisée en ce que la concentration en l'au moins un polyol [Po] est comprise entre 10 et 40 mg/g en poids total de ladite composition.

Dans un mode de réalisation, la composition destinée à être utilisée selon l'invention est caractérisée en ce que la concentration en l'au moins un polyol [Po] est comprise entre 15 et 30 mg/g en poids total de ladite composition.

Dans un mode de réalisation, la composition destinée à être utilisée selon l'invention est caractérisée en ce que la concentration en l'au moins un polyol [Po] est comprise entre 15 et 25 mg/g en poids total de ladite composition.

Dans un mode de réalisation, la composition destinée à être utilisée selon l'invention est caractérisée en ce que la concentration en l'au moins un polyol [Po] est comprise entre 20 et 40 mg/g en poids total de ladite composition.

Dans un mode de réalisation, la composition destinée à être utilisée selon l'invention est caractérisée en ce que la concentration en l'au moins un polyol [Po] est comprise entre 20 et 30 mg/g en poids total de ladite composition.

Dans un mode de réalisation, la composition destinée à être utilisée selon l'invention est caractérisée en ce que la concentration en l'au moins un polyol [Po] est comprise entre 25 et 35 mg/g en poids total de ladite composition.

Dans un mode de réalisation, la composition destinée à être utilisée selon l'invention est caractérisée en ce que la concentration en l'au moins un polyol [Po] est de 35 mg/g en poids total de ladite composition.

Dans un mode de réalisation, la composition destinée à être utilisée selon l'invention est caractérisée en ce que l'au moins un polyol est le mannitol et sa concentration est comprise entre 10 et 40 mg/g en poids total de ladite composition.

Dans un mode de réalisation, la composition destinée à être utilisée selon l'invention est caractérisée en ce que l'au moins un polyol est le mannitol et sa concentration est comprise entre 15 et 30 mg/g en poids total de ladite composition.

Dans un mode de réalisation, la composition destinée à être utilisée selon l'invention est caractérisée en ce que l'au moins un polyol est le mannitol et sa concentration est comprise entre 15 et 25 mg/g en poids total de ladite composition.

Dans un mode de réalisation, la composition destinée à être utilisée selon l'invention est caractérisée en ce que l'au moins un polyol est le mannitol et sa concentration est comprise entre 20 et 40 mg/g en poids total de ladite composition.

Dans un mode de réalisation, la composition destinée à être utilisée selon l'invention est caractérisée en ce que l'au moins un polyol est le mannitol et sa concentration est comprise entre 25 et 35 mg/g en poids total de ladite composition.

Dans un mode de réalisation, la composition destinée à être utilisée selon l'invention est caractérisée en ce que l'au moins un polyol est le mannitol et sa concentration est de 35 mg/g en poids total de ladite composition.

Dans un mode de réalisation, la composition destinée à être utilisée selon l'invention est caractérisée en ce que l'au moins un polyol est le sorbitol et sa concentration est comprise entre 10 et 40 mg/g en poids total de ladite composition.

Dans un mode de réalisation, la composition destinée à être utilisée selon l'invention est caractérisée en ce que l'au moins un polyol est le sorbitol et sa concentration est comprise entre 15 et 30 mg/g en poids total de ladite composition.

Dans un mode de réalisation, la composition destinée à être utilisée selon l'invention est caractérisée en ce que l'au moins un polyol est le sorbitol et sa concentration est comprise entre 15 et 25 mg/g en poids total de ladite composition.

Dans un mode de réalisation, la composition destinée à être utilisée selon l'invention est caractérisée en ce que l'au moins un polyol est le sorbitol et sa concentration est comprise entre 20 et 40 mg/g en poids total de ladite composition.

Dans un mode de réalisation, la composition destinée à être utilisée selon l'invention est caractérisée en ce que l'au moins un polyol est le sorbitol et sa concentration est comprise entre 25 et 35 mg/g en poids total de ladite composition.

Dans un mode de réalisation, la composition destinée à être utilisée selon l'invention est caractérisée en ce que l'au moins un polyol est le sorbitol et sa concentration est de 35 mg/g en poids total de ladite composition.

Dans un mode de réalisation, la composition destinée à être utilisée selon l'invention est caractérisée en ce que l'au moins un polyol est le maltitol et sa concentration est comprise entre 10 et 40 mg/g en poids total de ladite composition.

Dans un mode de réalisation, la composition destinée à être utilisée selon l'invention est caractérisée en ce que l'au moins un polyol est le maltitol et sa concentration est comprise entre 15 et 30 mg/g en poids total de ladite composition.

Dans un mode de réalisation, la composition destinée à être utilisée selon l'invention est caractérisée en ce que l'au moins un polyol est le maltitol et sa concentration est comprise entre 15 et 25 mg/g en poids total de ladite composition.

Dans un mode de réalisation, la composition destinée à être utilisée selon l'invention est caractérisée en ce que l'au moins un polyol est le maltitol et sa concentration est comprise entre 20 et 40 mg/g en poids total de ladite composition.

Dans un mode de réalisation, la composition destinée à être utilisée selon l'invention est caractérisée en ce que l'au moins un polyol est le maltitol et sa concentration est comprise entre 25 et 35 mg/g en poids total de ladite composition.

Dans un mode de réalisation, la composition destinée à être utilisée selon l'invention est caractérisée en ce que l'au moins un polyol est le maltitol et sa concentration est de 35 mg/g en poids total de ladite composition.

Dans un mode de réalisation, la composition destinée à être utilisée selon l'invention est caractérisée en ce que l'au moins un polyol est le glycérol et sa concentration est comprise entre 10 et 40 mg/g en poids total de ladite composition.

Dans un mode de réalisation, la composition destinée à être utilisée selon l'invention est caractérisée en ce que l'au moins un polyol est le glycérol et sa concentration est comprise entre 15 et 30 mg/g en poids total de ladite composition.

Dans un mode de réalisation, la composition destinée à être utilisée selon l'invention est caractérisée en ce que l'au moins un polyol est le glycérol et sa concentration est comprise entre 15 et 25 mg/g en poids total de ladite composition.

Dans un mode de réalisation, la composition destinée à être utilisée selon l'invention est caractérisée en ce que l'au moins un polyol est le glycérol et sa concentration est comprise entre 20 et 40 mg/g en poids total de ladite composition.

Dans un mode de réalisation, la composition destinée à être utilisée selon l'invention est caractérisée en ce que l'au moins un polyol est le glycérol et sa concentration est comprise entre 25 et 35 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO 2009 / 071697 au nom de la demanderesse, et la composition destinée à être utilisée selon l'invention est caractérisée en ce qu'elle comprend en outre au moins un polyol.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO 2009 / 071697 au nom de la demanderesse, et la composition destinée à être utilisée selon l'invention est caractérisée en ce que l'au moins un polyol est choisi dans le groupe constitué par le glycérol, le sorbitol, le propylène glycol, le xylitol, le mannitol, l'érythritol, le maltitol et le lactitol, seul ou en mélange.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est choisi dans le groupe constitué par le mannitol, le sorbitol, le maltitol et le glycérol, seul ou en mélange.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est choisi dans le groupe constitué par le mannitol, le sorbitol et le maltitol, seul ou en mélange.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le mannitol.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le sorbitol.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le maltitol.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le glycérol.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce qu'elle comprend au moins du mannitol et du sorbitol.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce qu'elle comprend au moins du mannitol et du maltitol.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que la concentration en l'au moins un polyol [Po] est comprise entre 0,01 mg/g et 50 mg/g.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que la concentration en l'au moins un polyol [Po] est comprise entre 10 et 40 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que la concentration en l'au moins un polyol [Po] est comprise entre 15 et 30 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que la concentration en l'au moins un polyol [Po] est comprise entre 15 et 25 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que la concentration en l'au moins un polyol [Po] est comprise entre 20 et 40 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que la concentration en l'au moins un polyol [Po] est comprise entre 20 et 30 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que la concentration en l'au moins un polyol [Po] est comprise entre 25 et 35 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que la concentration en l'au moins un polyol [Po] est de 35 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le mannitol et sa concentration est comprise entre 10 et 40 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le mannitol et sa concentration est comprise entre 15 et 30 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le mannitol et sa concentration est comprise entre 15 et 25 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le mannitol et sa concentration est comprise entre 20 et 40 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le mannitol et sa concentration est comprise entre 25 et 35 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le mannitol et sa concentration est de 35 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le sorbitol et sa concentration est comprise entre 10 et 40 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le sorbitol et sa concentration est comprise entre 15 et 30 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le sorbitol et sa concentration est comprise entre 15 et 25 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le sorbitol et sa concentration est comprise entre 20 et 40 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le sorbitol et sa concentration est comprise entre 25 et 35 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le sorbitol et sa concentration est de 35 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le maltitol et sa concentration est comprise entre 10 et 40 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le maltitol et sa concentration est comprise entre 15 et 30 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le maltitol et sa concentration est comprise entre 15 et 25 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le maltitol et sa concentration est comprise entre 20 et 40 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le maltitol et sa concentration est comprise entre 25 et 35 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le maltitol et sa concentration est de 35 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le glycérol et sa concentration est comprise entre 10 et 40 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le glycérol et sa concentration est comprise entre 15 et 30 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le glycérol et sa concentration est comprise entre 15 et 25 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le glycérol et sa concentration est comprise entre 20 et 40 mg/g en poids total de ladite composition.

Dans un mode de réalisation, le mélange d'acides hyaluroniques, ou de leurs sels, réticulés est un mélange monophasique de plusieurs acides hyaluroniques réticulés, tel que celui décrit dans la demande de brevet WO2009071697 au nom de la demanderesse, et la composition destinée à être utilisée selon l'invention est caractérisée en ce que l'au moins un polyol est le glycérol et sa concentration est comprise entre 25 et 35 mg/g en poids total de ladite composition.

Dans un mode de réalisation, la composition destinée à être utilisée selon l'invention est caractérisée en ce que l'au moins un polyol est le glycérol et sa concentration est de 35 mg/g en poids total de ladite composition.

Dans un mode de réalisation, la composition destinée à être utilisée selon l'invention est caractérisée en ce que le ratio massique entre la concentration en l'au moins un polyol [Po] et la concentration en mepivacaïne [MEPI] ; [Po] / [MEPI] est compris entre 0,0002 et 5000 ; 0,0002 ≤ [Po] / [MEPI] ≤ 5000.

Dans un mode de réalisation, la composition destinée à être utilisée selon l'invention est caractérisée en ce que le ratio massique entre la concentration en l'au moins un polyol [Po] et la concentration en mepivacaïne [MEPI] ; [Po] / [MEPI] est compris entre 0,002 et 500 ; 0,002 ≤ [Po] / [MEPI] ≤ 500.

Dans un mode de réalisation, la composition destinée à être utilisée selon l'invention est caractérisée en ce que le ratio massique entre la concentration en l'au moins un polyol [Po] et la concentration en mepivacaïne [MEPI] ; [Po] / [MEPI] est compris entre 0,02 et 50 ; 0,02 ≤ [Po] / [MEPI] ≤ 50.

Dans un mode de réalisation, la composition destinée à être utilisée selon l'invention est caractérisée en ce que le ratio massique entre la concentration en l'au moins un polyol [Po] et la concentration en mepivacaïne [MEPI] ; [Po] / [MEPI] est compris entre 1 et 20 ; 1 ≤ [Po] / [MEPI] ≤ 20.

Dans un mode de réalisation, la composition destinée à être utilisée selon l'invention est caractérisée en ce que le ratio massique entre la concentration en l'au moins un polyol [Po] et la concentration en mepivacaïne [MEPI] ; [Po] / [MEPI] est compris entre 3 et 15 ; 3 ≤ [Po] / [MEPI] ≤ 15.

Dans un mode de réalisation, la composition destinée à être utilisée selon l'invention est caractérisée en ce que le ratio massique entre la concentration en l'au moins un polyol [Po] et la concentration en mepivacaïne [MEPI] ; [Po] / [MEPI] est compris entre 4 et 8 ; 4 ≤ [Po] / [MEPI] ≤ 8.

Dans un mode de réalisation, la composition destinée à être utilisée selon l'invention est caractérisée en ce que le ratio massique entre la concentration en l'au moins un polyol [Po] et la concentration en mepivacaïne [MEPI] ; [Po] / [MEPI] est compris entre 10 et 13 ; 10 ≤ [Po] / [MEPI] ≤ 13.

Dans un mode de réalisation, la composition destinée à être utilisée selon l'invention est caractérisée en ce qu'elle comprend en outre au moins un second anesthésique local.

Dans un mode de réalisation, la composition destinée à être utilisée selon l'invention est caractérisée en ce que l'au moins un second anesthésique local est choisi dans le groupe constitué par la lidocaïne, la bupivacaïne, la benzocaïne, la chloroprocaïne, la procaïne, l'étidocaïne, l'aptocaïne, le chlorobutanol, la diamocaïne, la dyclonine, le guafécaïnol, le polidocanol, l'articaïne, la bupivacaïne, la ropivacaïne, la tétracaïne, la pramocaïne et leurs sels et leurs isomères isolés.

Dans un mode de réalisation, la composition destinée à être utilisée selon l'invention est caractérisée en ce que l'au moins un second anesthésique local est un amino-amide.

Dans un mode de réalisation, la composition destinée à être utilisée selon l'invention est caractérisée en ce que l'au moins un second anesthésique local est un amino-amide choisi dans le groupe constitué par la lidocaïne, l'étidocaïne, l'aptocaïne, l'articaïne, la bupivacaïne, la ropivacaïne et leurs sels et leurs isomères isolés.

Dans un mode de réalisation, la composition destinée à être utilisée selon l'invention est caractérisée en ce que l'au moins un second anesthésique local est un amino-ester.

Dans un mode de réalisation, la composition destinée à être utilisée selon l'invention est caractérisée en ce que l'au moins un second anesthésique local est un amino-esters choisi dans le groupe constitué par par la benzocaïne, la chloroprocaïne, la procaïne, la tétracaïne et leurs sels et leurs isomères isolés.

Dans un mode de réalisation, la composition destinée à être utilisée selon l'invention est caractérisée en ce que l'au moins un second anesthésique local est choisi dans le groupe constitué par le chlorobutanol, la dyclonine, le polidocanol et leurs sels et leurs isomères isolés.

Dans un mode de réalisation, la composition destinée à être utilisée selon l'invention est caractérisée en ce que la concentration en anesthésique(s) local (locaux) [AL] est comprise entre 0,01 mg/g et 50 mg/g de poids total de ladite composition.

Dans un mode de réalisation, la composition destinée à être utilisée selon l'invention est caractérisée en ce que la concentration en anesthésique(s) local (locaux) [AL] est comprise entre 0,05 mg/g et 45 mg/g de poids total de ladite composition.

Dans un mode de réalisation, la composition destinée à être utilisée selon l'invention est caractérisée en ce que la concentration en anesthésique(s) local (locaux) [AL] est comprise entre 0,1 mg/g et 40 mg/g de poids total de ladite composition.

Dans un mode de réalisation, la composition destinée à être utilisée selon l'invention est caractérisée en ce que la concentration en anesthésique(s) local (locaux) [AL] est comprise entre 0,2 mg/g et 30 mg/g de poids total de ladite composition.

Dans un mode de réalisation, la composition destinée à être utilisée selon l'invention est caractérisée en ce que la concentration en anesthésique(s) local (locaux) [AL] est comprise entre 0,5 mg/g et 20 mg/g de poids total de ladite composition.

Dans un mode de réalisation, la composition destinée à être utilisée selon l'invention est caractérisée en ce que la concentration en anesthésique(s) local (locaux) [AL] est comprise entre 1 mg/g et 15 mg/g de poids total de ladite composition.

Dans un mode de réalisation, la composition destinée à être utilisée selon l'invention est caractérisée en ce que la concentration en anesthésique(s) local (locaux) [AL] est comprise entre 1 mg/g et 10 mg/g de poids total de ladite composition.

Dans un mode de réalisation, la composition destinée à être utilisée selon l'invention est caractérisée en ce que la concentration en anesthésique(s) local (locaux) [AL] est comprise entre 1 mg/g et 6 mg/g de poids total de ladite composition.

Dans un mode de réalisation, la composition destinée à être utilisée selon l'invention est caractérisée en ce que la concentration en anesthésique(s) local (locaux) [AL] est comprise entre 1 mg/g et 5 mg/g de poids total de ladite composition.

Dans un mode de réalisation, la composition destinée à être utilisée selon l'invention est caractérisée en ce que la concentration en anesthésique(s) local (locaux) [AL] est comprise entre 2 mg/g et 5 mg/g de poids total de ladite composition.

Dans un mode de réalisation, la composition destinée à être utilisée selon l'invention est caractérisée en ce que la concentration en anesthésique(s) local (locaux) [AL] est comprise entre 6 mg/g et 10 mg/g de poids total de ladite composition.

Dans un mode de réalisation, la composition destinée à être utilisée selon l'invention est caractérisée en ce que la concentration en anesthésique(s) local (locaux) [AL] est de 1 mg/g de poids total de ladite composition.

Dans un mode de réalisation, la composition destinée à être utilisée selon l'invention est caractérisée en ce que la concentration en anesthésique(s) local (locaux) [AL] est de 3 mg/g de poids total de ladite composition.

Dans un mode de réalisation, la composition destinée à être utilisée selon l'invention est caractérisée en ce que concentration en l'au moins un anesthésique local [AL] est de 4 mg/g de poids total de ladite composition.

Dans un mode de réalisation, la composition destinée à être utilisée selon l'invention est caractérisée en ce que la concentration en anesthésique(s) local (locaux) [AL] est de 5 mg/g de poids total de ladite composition.

Dans un mode de réalisation, la composition destinée à être utilisée selon l'invention est caractérisée en ce que la concentration en anesthésique(s) local (locaux) [AL] est de 6 mg/g de poids total de ladite composition.

Dans un mode de réalisation, la composition destinée à être utilisée selon l'invention est caractérisée en ce que la concentration en anesthésique(s) local (locaux) [AL] est de 10 mg/g de poids total de ladite composition.

Dans un mode de réalisation, la composition destinée à être utilisée selon l'invention est caractérisée en ce que le ratio massique entre la concentration en l'au moins un acide hyaluronique [HA] et la concentration en anesthésique(s) local (locaux) [AL] ; [HA] / [AL] est compris entre 0,1 et 50 ; 0,1 ≤ [HA] / [AL] ≤ 50.

Dans un mode de réalisation, la composition destinée à être utilisée selon l'invention est caractérisée en ce que le ratio massique entre la concentration en l'au moins un acide hyaluronique [HA] et la concentration en anesthésique(s) local (locaux) [AL] : [HA] / [AL] est compris entre 0,5 et 40, 0,5 ≤ [HA] / [AL] ≤ 40.

Dans un mode de réalisation, la composition destinée à être utilisée selon l'invention est caractérisée en ce que le ratio massique entre la concentration en l'au moins un acide hyaluronique [HA] et la concentration en anesthésique(s) local (locaux) [AL] : [HA] / [AL] est compris entre 1 et 30, 1 ≤ [HA] / [AL] ≤ 30.

Dans un mode de réalisation, la composition destinée à être utilisée selon l'invention est caractérisée en ce que le ratio massique entre la concentration en l'au moins un acide hyaluronique [HA] et la concentration en anesthésique(s) local (locaux) [AL] : [HA] / [AL] est compris entre 2 et 20, 2 ≤ [HA] / [AL] ≤ 20.

Dans un mode de réalisation, la composition destinée à être utilisée selon l'invention est caractérisée en ce que le ratio massique entre la concentration en l'au moins un acide hyaluronique [HA] et la concentration en anesthésique(s) local (locaux) [AL] : [HA] / [AL] est compris entre 7 / 3 et 26 / 3, 7 / 3 ≤ [HA] / [AL] ≤ 26 / 3.

Dans un mode de réalisation, la composition destinée à être utilisée selon l'invention est caractérisée en ce que le ratio massique entre la concentration en l'au moins un acide hyaluronique [HA] et la concentration en anesthésique(s) local (locaux) [AL] : [HA] / [AL] est compris entre 2 et 20 / 3, 2 ≤ [HA] / [AL] ≤ 20 / 3.

Dans un mode de réalisation, la composition destinée à être utilisée selon l'invention est caractérisée en ce que le ratio massique entre la concentration en l'au moins un acide hyaluronique [HA] et la concentration en anesthésique(s) local (locaux) [AL] : [HA] / [AL] est compris entre 2 et 10 / 3, 2 ≤ [HA] / [AL] ≤ 10 / 3.

Dans un mode de réalisation, la composition destinée à être utilisée selon l'invention est caractérisée en ce que le ratio massique entre la concentration en l'au moins un acide hyaluronique [HA] et la concentration en anesthésique(s) local (locaux) [AL] : [HA] / [AL] est de 20.

Dans un mode de réalisation, la composition destinée à être utilisée selon l'invention est caractérisée en ce que le ratio massique entre la concentration en l'au moins un acide hyaluronique [HA] et la concentration en anesthésique(s) local (locaux) [AL] : [HA] / [AL] est de 26 / 3.

Dans un mode de réalisation, la composition destinée à être utilisée selon l'invention est caractérisée en ce que le ratio massique entre la concentration en l'au moins un acide hyaluronique [HA] et la concentration en anesthésique(s) local (locaux) [AL] : [HA] / [AL] est de 20 / 3.

Dans un mode de réalisation, la composition destinée à être utilisée selon l'invention est caractérisée en ce que le ratio massique entre la concentration en l'au moins un acide hyaluronique [HA] et la concentration en anesthésique(s) local (locaux) [AL] : [HA] / [AL] est de 10 / 3.

Dans un mode de réalisation, la composition destinée à être utilisée selon l'invention est caractérisée en ce que le ratio massique entre la concentration en l'au moins un acide hyaluronique [HA] et la concentration en anesthésique(s) local (locaux) [AL] : [HA] / [AL] est de 7 / 3.

Dans un mode de réalisation, la composition destinée à être utilisée selon l'invention est caractérisée en ce que le ratio massique entre la concentration en l'au moins un acide hyaluronique [HA] et la concentration en anesthésique(s) local (locaux) [AL] : [HA] / [AL] est de 2.

Dans un mode de réalisation, la composition destinée à être utilisée selon l'invention est caractérisée en ce que le ratio massique entre la concentration en l'au moins un polyol [Po] et la concentration en anesthésique(s) local (locaux) [AL] ; [Po] / [AL] est compris entre 0,0002 et 5000 ; 0,0002 ≤ [Po] / [AL] ≤ 5000.

Dans un mode de réalisation, la composition destinée à être utilisée selon l'invention est caractérisée en ce que le ratio massique entre la concentration en l'au moins un polyol [Po] et la concentration en anesthésique(s) local (locaux) [AL] ; [Po] / [AL] est compris entre 0,002 et 500 ; 0,002 ≤ [Po] / [AL] ≤ 500.

Dans un mode de réalisation, la composition destinée à être utilisée selon l'invention est caractérisée en ce que le ratio massique entre la concentration en l'au moins un polyol [Po] et la concentration en anesthésique(s) local (locaux) [AL] ; [Po] / [AL] est compris entre 0,02 et 50 ; 0,02 ≤ [Po] / [AL] ≤ 50.

Dans un mode de réalisation, la composition destinée à être utilisée selon l'invention est caractérisée en ce que le ratio massique entre la concentration en l'au moins un polyol [Po] et la concentration en anesthésique(s) local (locaux) [AL] ; [Po] / [AL] est compris entre 1 et 20 ; 1 ≤ [Po] / [AL] ≤ 20.

Dans un mode de réalisation, la composition destinée à être utilisée selon l'invention est caractérisée en ce que le ratio massique entre la concentration en l'au moins un polyol [Po] et la concentration en anesthésique(s) local (locaux) [AL] ; [Po] / [AL] est compris entre 3 et 15 ; 3 ≤ [Po] / [AL] ≤ 15.

Dans un mode de réalisation, la composition destinée à être utilisée selon l'invention est caractérisée en ce que le ratio massique entre la concentration en l'au moins un polyol [Po] et la concentration en anesthésique(s) local (locaux) [AL] ; [Po] / [AL] est compris entre 4 et 8 ; 4 ≤ [Po] / [AL] ≤ 8.

Dans un mode de réalisation, la composition destinée à être utilisée selon l'invention est caractérisée en ce que le ratio massique entre la concentration en l'au moins un polyol [Po] et la concentration en anesthésique(s) local (locaux) [AL] ; [Po] / [AL] est compris entre 10 et 13 ; 10 ≤ [Po] / [AL] ≤ 13.

Dans un mode de réalisation, la composition destinée à être utilisée selon l'invention est caractérisée en ce que ladite composition est stérilisée.

Dans un mode de réalisation, la composition destinée à être utilisée selon l'invention est caractérisée en ce que la stérilisation est effectuée par la chaleur, la chaleur humide (et en particulier l'autoclavage à la vapeur), le rayonnement gamma (γ), ou par faisceau d'électrons accélérés (Electron-beam).

Dans un mode de réalisation, la composition destinée à être utilisée selon l'invention est caractérisée en ce que la stérilisation est effectuée par la chaleur.

Dans un mode de réalisation, la composition destinée à être utilisée selon l'invention est caractérisée en ce que la stérilisation est effectuée par la chaleur humide.

Dans un mode de réalisation, la composition destinée à être utilisée selon l'invention est caractérisée en ce que la stérilisation est effectuée par autoclavage à la vapeur.

Dans un mode de réalisation, la composition destinée à être utilisée selon l'invention est caractérisée en ce que la stérilisation par autoclavage à la vapeur est réalisée à une température de 121 à 134°C, pendant une durée adaptée à la température.

Dans un mode de réalisation, la composition destinée à être utilisée selon l'invention est caractérisée en ce que la stérilisation par autoclavage à la vapeur est réalisée à une température comprise entre 127 et 130°C pendant une durée comprise entre 1 et 20 min.

Dans un mode de réalisation, la composition destinée à être utilisée selon l'invention est caractérisée en ce que la stérilisation est effectuée par irradiation par rayonnements gamma (γ).

Dans un mode de réalisation, la composition destinée à être utilisée selon l'invention est caractérisée en ce qu'elle est appliquée par injection(s) esthétique(s) au niveau du visage.

Dans un mode de réalisation, la composition destinée à être utilisée selon l'invention est caractérisée en ce qu'elle est appliquée par injection(s) esthétique(s) au niveau du corps.

Dans un mode de réalisation, la composition destinée à être utilisée selon l'invention est caractérisée en ce qu'elle est appliquée pour le traitement de l'arthrose.

Les applications visées sont plus particulièrement les applications communément répandues dans le cadre des viscoélastiques injectables et des polysaccharides utilisés ou potentiellement utilisables dans les pathologies ou traitements suivants :
- injections esthétiques au niveau du visage : de comblement de rides, défauts cutanés ou volumatrices (pommettes, menton, lèvres) ;
- injections volumatrices au niveau du corps : augmentation des seins et des fesses, augmentation du point G, vaginoplastie, reconstruction des lèvres vaginales, augmentation de la taille du pénis ;
- traitement de l'arthrose, injection dans l'articulation en remplacement ou en complément du liquide synovial déficient ;
- injection péri-urétrale pour le traitement de l'incontinence urinaire par insuffisance sphinctérienne ;
- injection post-chirurgicale pour éviter les adhésions péritonéales notamment ;
- injection suite à une chirurgie de la presbytie par incisions sclérales au laser ;
- injection dans la cavité vitréenne ;
- injection au cours de la chirurgie de la cataracte ;
- injection dans les parties génitales.

Plus particulièrement, en chirurgie esthétique, en fonction de ses propriétés viscoélastiques et de rémanence, la composition destinée à être utilisée selon l'invention pourra être utilisée :
- pour le comblement des rides fines, moyennes ou profondes, et être injectée avec des aiguilles de diamètre fin (27 Gauge par exemple) ;
- comme volumateur avec une injection par des aiguilles de diamètre plus important, de 22 à 26 Gauge par exemple, et plus longues (30 à 40 mm par exemple); dans ce cas, son caractère cohésif permettra de garantir son maintien à l'emplacement de l'injection.
- La composition destinée à être utilisée selon l'invention trouve également une application importante en chirurgie articulaire et en chirurgie dentaire pour le comblement des poches parodontales par exemple.

Ces exemples d'utilisation ne sont nullement limitatifs, la composition destinée à être utilisée selon la présente invention étant plus largement prévue pour :
- combler des volumes ;
- générer des espaces au sein de certains tissus, favorisant ainsi leur fonctionnement optimal ;
- remplacer des liquides physiologiques déficients.

Dans un mode de réalisation, la composition destinée à être utilisée selon l'invention est caractérisée en ce qu'elle comprend en outre au moins un composé additionnel.

Dans un mode de réalisation, la composition destinée à être utilisée selon l'invention est caractérisée en ce que la concentration en l'au moins un composé additionnel [CA] est comprise entre 0,1 et 100 mg/g de poids total de ladite composition.

Dans un mode de réalisation, la composition destinée à être utilisée selon l'invention est caractérisée en ce que la concentration en l'au moins un composé additionnel [CA] est comprise entre 1 et 50 mg/g de poids total de ladite composition.

Dans un mode de réalisation, la composition destinée à être utilisée selon l'invention est caractérisée en ce que l'au moins un composé additionnel est la diméthyl sulfone, ci-après DMS.

Dans un mode de réalisation, la composition destinée à être utilisée selon l'invention est caractérisée en ce que l'au moins un composé additionnel est un sel hydrosoluble de sucrose octasulfate, ci-après SOS.

Dans un mode de réalisation, la composition destinée à être utilisée selon l'invention est caractérisée en ce que l'au moins un composé additionnel est un dérivé de vitamine C.

Dans un mode de réalisation, la composition destinée à être utilisée selon l'invention est caractérisée en ce que l'au moins un composé additionnel est un sel d'ascorbyl phosphate de magnésium, ci-après MAP.

Dans un mode de réalisation, la composition destinée à être utilisée selon l'invention est caractérisée en ce que l'au moins un composé additionnel appartient à la famille des catécholamines.

Dans un mode de réalisation, la composition destinée à être utilisée selon l'invention est caractérisée en ce que l'au moins un composé additionnel appartenant à la famille des catécholamines, est l'épinéphrine.

Dans un mode de réalisation, la composition destinée à être utilisée selon l'invention est caractérisée en ce que la concentration en l'au moins un composé additionnel [CA] est comprise entre 0,01 et 10 % en poids par rapport au poids total de ladite composition.

Dans un mode de réalisation, la composition destinée à être utilisée selon l'invention est caractérisée en ce que la concentration en l'au moins un composé additionnel [CA] est comprise entre 0,1 et 5 % en poids par rapport au poids total de ladite composition.

Dans un mode de réalisation, la composition destinée à être utilisée selon l'invention est caractérisée en ce que l'au moins un composé additionnel est la diméthyl sulfone et sa concentration est comprise entre 1 et 10 mg/g en poids total de ladite composition.

Dans un mode de réalisation, la composition destinée à être utilisée selon l'invention est caractérisée en ce que l'au moins un composé additionnel est un sel hydrosoluble de sucrose octasulfate et sa concentration est comprise entre 1 et 40 mg/g en poids total de ladite composition.

Dans un mode de réalisation, la composition destinée à être utilisée selon l'invention est caractérisée en ce que l'au moins un composé additionnel est un sel d'ascorbyl phosphate de magnésium et sa concentration est comprise entre 0,3 et 20 mg/g en poids total de ladite composition.

Dans un mode de réalisation, la composition destinée à être utilisée selon l'invention est caractérisée en ce qu'elle est injectable.

L'invention concerne également une méthode de prévention de la formation des oedèmes, caractérisée en ce qu'elle comprend l'administration d'une composition aqueuse comprenant au moins un acide hyaluronique et de la mépivacaïne.

L'invention concerne également une méthode de prévention de la formation des oedèmes induits par une composition injectable comprenant de l'acide hyaluronique, caractérisée en ce que ladite composition comprend en outre de la mépivacaïne.

L'invention concerne également l'utilisation d'une composition aqueuse comprenant au moins un acide hyaluronique et de la mépivacaïne pour la prévention de la formation des oedèmes.

L'invention concerne également l'utilisation de la mépivacaïne dans une composition injectable comprenant de l'acide hyaluronique, caractérisée en ce que ladite mépivacaïne prévient la formation des oedèmes induits par ladite composition.
Tous les modes de réalisation applicables à la composition aqueuse destinée à être utilisée selon l'invention sont également applicables aux utilisations d'une composition aqueuse selon l'invention, ou bien encore aux méthodes de prévention de la formation des oedèmes selon l'invention.

Exemple 1 : Effets d'une composition « acide hyaluronique + mépivacaïne + mannitol » selon l'invention par rapport à une composition « acide hyaluronique + lidocaïne + mannitol », eu égard à l'œdème.

Deux types de composition aqueuse à base d'acide hyaluronique (concentration 20 mg/g) sont préparés.

Toutes les compositions sont issues d'un mélange monophasique tel que celui décrit dans la demande de brevet WO 2009/071697 au nom de la demanderesse, et comprennent du mannitol (concentration 35 mg/g).

Leurs compositions diffèrent en ce qui concerne l'anesthésique local, qui est soit de la mépivacaïne (concentration 3 mg/g), soit de la lidocaïne (concentration 3 mg/g).

Deux groupes A et B de 64 patients chacun sont formés (128 patients au total).

Le groupe A est injecté au niveau du sillon naso-génien au moyen de la composition aqueuse à base d'acide hyaluronique comprenant du mannitol et de la lidocaïne.

Le groupe B est injecté au niveau du sillon naso-génien au moyen de la composition aqueuse à base d'acide hyaluronique comprenant du mannitol et de la mépivacaïne.

Trois jours après l'injection (à J3), pour chaque sujet demandeur des deux groupes A (injecté au niveau du sillon naso-génien au moyen de la composition aqueuse à base d'acide hyaluronique comprenant du mannitol et de la lidocaïne) et B (injecté au niveau du sillon naso-génien au moyen de la composition aqueuse à base d'acide hyaluronique comprenant du mannitol et de la mépivacaïne), la personne en charge de l'administration évalue l'occurrence et la sévérité de l'oedème et de l'ecchymose, selon l'échelle suivante :
- pour l'oedème : aucun, léger, modéré, sévère ;
- pour l'ecchymose : aucune, légère, modérée, sévère.

Les résultats sont repris dans le tableau 1 ci-après :

**Tableau 1**

| **Paramètre évalué à J3** | **Évaluation de la personne en charge de l'administration** | **Groupe A (lidocaïne)** | **Groupe B (mépivacaïne)** |
|---|---|---|---|
| Œdème | Aucun | 57 | 63 |
| | Léqer ou modéré | 7 | 1 |
| | Sévère | 0 | 0 |
| Ecchymose | Aucune | 43 | 42 |
| | Légère ou modérée | 21 | 22 |
| | Sévère | 0 | 0 |

Il est déductible de ce tableau que l'association « mépivacaïne + mannitol » est bien meilleure du point de vue de l'œdème (pas d'oedème ou oedème moins prononcé) que l'association « lidocaïne + mannitol ».

En revanche, aucune différence significative n'est observée s'agissant de l'ecchymose.

## Revendications

1. Mépivacaïne pour utilisation dans la prévention de la formation des oedèmes induits par une composition aqueuse comprenant au moins un acide hyaluronique, ladite composition comprenant ladite mépivacaïne.

2. Mépivacaïne pour utilisation selon la revendication 1, **caractérisée en ce qu'**elle comprend au moins un acide hyaluronique non réticulé ou l'un de ses sels, seul ou en mélange.

3. Mépivacaïne pour utilisation selon la revendication 2, **caractérisée en ce qu'**elle comprend un mélange d'acides hyaluroniques, non réticulés.

4. Mépivacaïne pour utilisation selon la revendication 1, **caractérisée en ce qu'**elle comprend au moins un acide hyaluronique réticulé ou l'un de ses sels, seul ou en mélange.

5. Mépivacaïne pour utilisation selon la revendication 4, **caractérisée en ce qu'**elle comprend un mélange d'acides hyaluroniques, réticulés.

6. Mépivacaïne pour utilisation selon l'une quelconque des revendications précédentes, **caractérisée en ce qu'**elle comprend un mélange d'acides hyaluroniques, réticulé(s) et non réticulé(s).

7. Mépivacaïne pour utilisation selon l'une quelconque des revendications précédentes, **caractérisée en ce qu'**elle comprend en outre au moins un polyol.

8. Mépivacaïne pour utilisation selon l'une quelconque des revendications précédentes, **caractérisée en ce qu'**elle comprend en outre au moins un second anesthésique local.

9. Mépivacaïne pour utilisation selon l'une quelconque des revendications précédentes, **caractérisée en ce que** ladite composition est stérilisée.

10. Mépivacaïne pour utilisation selon l'une quelconque des revendications précédentes, **caractérisée en ce qu'**elle est appliquée par injection(s) esthétique(s) au niveau du visage.

11. Mépivacaïne pour utilisation selon l'une quelconque des revendications 1 à 9, **caractérisée en ce qu'**elle est appliquée par injection(s) esthétique(s) au niveau du corps.

12. Mépivacaïne pour utilisation selon l'une quelconque des revendications précédentes, **caractérisée en ce que** ladite composition comprend au moins un composé additionnel.

13. Mépivacaïne pour utilisation selon l'une quelconque des revendications précédentes, **caractérisée en ce qu'**elle est injectable.

## Patentansprüche

1. Mepivacain zur Verwendung beim Verhindern von durch eine Zusammensetzung umfassend mindestens eine Hyaluronsäure induzierten Ödemen, wobei die Zusammensetzung das Mepivacain umfasst.

2. Mepivacain zur Verwendung nach Anspruch 1, **dadurch gekennzeichnet, dass** sie mindestens eine nicht-vernetzte Hyaluronsäure oder eines ihrer Salze umfasst, entweder einzeln oder als Mischung.

3. Mepivacain zur Verwendung nach Anspruch 2, **dadurch gekennzeichnet, dass** sie eine Mischung aus nicht-vernetzten Hyaluronsäuren umfasst.

4. Mepivacain zur Verwendung nach Anspruch 1, **dadurch gekennzeichnet, dass** sie mindestens eine vernetzte Hyaluronsäure oder eines ihrer Salze umfasst, entweder einzeln oder als Mischung.

5. Mepivacain zur Verwendung nach Anspruch 4, **dadurch gekennzeichnet, dass** sie eine Mischung aus vernetzten Hyaluronsäuren umfasst.

6. Mepivacain zur Verwendung nach einem der vorangehenden Ansprüche, **dadurch gekennzeichnet, dass** sie eine Mischung aus vernetzten und nicht-vernetzten Hyaluronsäuren umfasst.

7. Mepivacain zur Verwendung nach einem der vorangehenden Ansprüche, **dadurch gekennzeichnet, dass** sie zusätzlich mindestens ein Polyol umfasst.

8. Mepivacain zur Verwendung nach einem der vorangehenden Ansprüche, **dadurch gekennzeichnet, dass** sie zusätzlich mindestens ein zweites Lokalanästhetikum umfasst.

9. Mepivacain zur Verwendung nach einem der vorangehenden Ansprüche, **dadurch gekennzeichnet, dass** die Zusammensetzung sterilisiert ist.

10. Mepivacain zur Verwendung nach einem der vorangehenden Ansprüche, **dadurch gekennzeichnet, dass** sie durch ästhetische Injektion(en) im Gesicht verabreicht wird.

11. Mepivacain zur Verwendung nach einem der Ansprüche 1 bis 9, **dadurch gekennzeichnet, dass** sie durch ästhetische Injektion(en) am Körper verabreicht wird.

12. Mepivacain zur Verwendung nach einem der vorangehenden Ansprüche, **dadurch gekennzeichnet, dass** die Zusammensetzung mindestens eine weitere Verbindung umfasst.

13. Mepivacain zur Verwendung nach einem der vorangehenden Ansprüche, **dadurch gekennzeichnet, dass** sie injizierbar ist.

## Claims

1. Mepivacaine for use in preventing the formation of edemas induced by an aqueous composition comprising at least one hyaluronic acid, said composition comprising the said mepivacaine.

2. Mepivacaine for use as set forth in claim 1, **characterized in that** it comprises at least one non-crosslinked hyaluronic acid or a salt thereof, alone or as a mixture.

3. Mepivacaine for use as set forth in claim 2, **characterized in that** it comprises a mixture of non-crosslinked hyaluronic acids.

4. Mepivacaine for use as set forth in claim 1, **characterized in that** it comprises at least one crosslinked hyaluronic acid or a salt thereof, alone or as a mixture.

5. Mepivacaine for use as set forth in claim 4, **characterized in that** it comprises a mixture of crosslinked hyaluronic acids.

6. Mepivacaine for use as set forth in any one of the preceding claims, **characterized in that** it comprises a mixture of crosslinked and non-crosslinked hyaluronic acids.

7. Mepivacaine as set forth in any one of the preceding claims, **characterized in that** it further comprises at least one polyol.

8. Mepivacaine as set forth in any one of the preceding claims, **characterized in that** it further comprises at least one second local anesthetic.

9. Mepivacaine for use as set forth in any one of the preceding claims, **characterized in that** said composition is sterilized.

10. Mepivacaine for use as set forth in any one of the preceding claims, **characterized in that** it is applied by esthetic injection(s) in the face.

11. Mepivacaine for use as set forth in any one of claims 1 to 9, **characterized in that** it is applied by esthetic injection(s) in the body.

12. Mepivacaine for use as set forth in any one of the preceding claims, **characterized in that** the said composition comprises at least one additional compound.

13. Mepivacaine for use as set forth in any one of the preceding claims, **characterized in that** it is injectable.
